## Europäisches Patentamt

## European Patent Office

## Office européen des brevets

(11) Veröffentlichungsnummer: **0 197 348**
**B1**

(12)

# EUROPÄISCHE PATENTSCHRIFT

(45) Veröffentlichungstag der Patentschrift:
22.11.90

(51) Int. Cl.⁵: **C10L 1/02**, C07C 41/06

(21) Anmeldenummer: **86103379.3**

(22) Anmeldetag: **13.03.86**

(54) **Alkyl-tert.-alkyl-ether enthaltende Gum-freie Kraftstoff-Komponenten.**

(30) Priorität: 23.03.85 DE 3510683
30.10.85 DE 3538564

(43) Veröffentlichungstag der Anmeldung:
15.10.86 Patentblatt 86/42

(45) Bekanntmachung des Hinweises auf die Patenterteilung:
22.11.90 Patentblatt 90/47

(84) Benannte Vertragsstaaten:
BE DE FR GB IT NL

(56) Entgegenhaltungen:
EP-A- 0 043 986

(73) Patentinhaber: EC ERDÖLCHEMIE GMBH,
Postfach 75 20 02, D-5000 Köln 71(DE)

(72) Erfinder: Köhler, Hans-Dieter, Dr., Stürzelberger
Strasse 71, D-4047 Dormagen 5(DE)
Erfinder: Schleppinghoff, Bernhard, Dr.,
Adolf-Kolpingstrasse 5, D-4047 Dormagen 1(DE)
Erfinder: Schulwitz, Bruno, Dr., An der Ronne 86,
D-5000 Koeln 40(DE)
Erfinder: Scheef, Hans-Volker, Goethe-Strasse 69,
D-4047 Dormagen 1(DE)
Erfinder: Tschorn, Herbert, Zonserstrasse 39,
D-4047 Dormagen 1(DE)

(74) Vertreter: Müller, Gerhard, Dr. et al, BAYER AG
Konzernverwaltung RP Patentabteilung,
D-5090 Leverkusen 1 Bayerwerk(DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft Gum-freie Kraftstoff-Komponenten mit einem Gehalt an Alkyl-tert.-alkyl-ethern, herstellbar aus rohen Kohlenwasserstoffgemischen, die tert.-Olefine enthalten.

Unter Gum wird im Zusammenhang mit Kraftstoffen ein Gehalt an oligomeren oder polymeren Stoffen im Kraftstoff verstanden, der bei der Analytik der Kraftstoffe als Abdampfrückstand zu Tage tritt. Ein Gehalt an Gum im Kraftstoff führt zu Verkokungen und Ablagerungen im Brennraum des Motors und ist daher äußerst unerwünscht.

Die Herstellung von Alkyl-tert.-alkylethern aus tert.-Olefinen und Alkanolen in Gegenwart von makroporösen oder gelförmigen sauren Kationenaustauschern in der H+-Form, wobei die Kationenaustauscher mit 0,1 bis 5 g eines Metalles der VI., VII. oder VIII. Nebengruppe des Periodensystems der Elemente in elementarer Form pro 1 Liter Kationenaustauscher belegt worden sind, ist aus EP 43 986 bekannt; diese Schrift beschreibt den Einsatz von tert.- Olefinen enthaltenden Raffinaten, eine Behandlung von Gum-Bildnern ist dieser Schrift nicht zu entnehmen.

Es wurden nun Alkyl-tert.-alkyl-ether enthaltende Gum-freie Kraftstoff-Komponenten gefunden, die dadurch herstellbar sind, daß man tert.-Olefine und Gum-Bildner enthaltende rohe Kohlenwasserstoffgemische gleichzeitig mit Alkanolen und Wasserstoff, der in einer Menge eingesetzt wird, die zur Gesamtmenge der Gum-Bildner mindestens äquimolar ist, an einem makroporösen oder gelförmigen Kationenaustauscher in der H+-Form, der 0,001 bis 10 g eines oder mehrerer Metalle der VI. und/oder VII. und/oder VIII. Nebengruppe des Periodensystems der Elemente in elementarer Form pro Liter trockenen Kationenaustauschers enthält und einen Vernetzungsgrad von 2-65 % sowie eine spezifische Oberfläche von 5-750 m²/g trockenes Austauscherharz aufweist, in flüssiger Phase bei 30-140°C umsetzt.

Als tert.-Olefine seien beispielsweise solche genannt, die 5-8, bevorzugt 5-7 C-Atome enthalten; besonders bevorzugt sind $C_5$- und/oder $C_6$-tert.-Olefine. Solche tert.-Olefine sind in den erfindungsgemäß einzusetzenden rohen Kohlenwasserstoffgemischen im wesentlichen als Gemisch mehrerer von ihnen enthalten. Beispielsweise enthält ein Kohlenwasserstoffgemisch, das im wesentlichen tert.-Amylene enthält, im allgemeinen geringe Mengen an i-Buten und/oder tert.-Hexenen.

Die erfindungsgemäß einzusetzenden rohen Kohlenwasserstoffgemische enthalten neben den beschriebenen tert.-Olefinen weiterhin geradkettige und/oder verzweigte und/oder cyclische gesättigte und einfach ungesättigte Kohlenwasserstoffe mit im wesentlichen 5-8 C-Atomen und geringen Mengen der anschließenden Homologen, beispielsweise geringeren Mengen an $C_4$-Kohlenwasserstoffen.

Weiterhin enthalten solche rohen Kohlenwasserstoffgemische Bestandteile, die unter den Herstellungsbedingungen oligomere oder polymere Stoffe bilden, die dem Fachmann als "Gum" bekannt sind. Bei diesen Gum-bildenden Bestandteilen handelt es sich beispielsweise um Diolefine und/oder Acetylen-Verbindungen. Bei engeren Kohlenwasserstoff-Schnitten (z.B. $C_5$ oder $C_6$) befinden sich außer den tert.-Olefinen auch die gesättigten und olefinischen Kohlenwasserstoffe sowie die Gum-Bildner hauptsächlich im Bereich der C-Zahl dieses Schnittes.

Solche Kohlenwasserstoffgemische mit verschiedenen C-Atomzahlen und unterschiedlichem Grad an Ungesättigtheit sowie dem Gehalt an tert.-Olefinen stehen in petrochemischen Anlagen oder Raffinerien zu Verfügung.

Diese Kohlenwasserstoffgemische können beispielsweise bei der Umsetzung von Naphtha, Liquid Petroleum Gas (LPG), Rohöldestillaten, Gasöl oder anderen Ausgangskohlenwasserstoffgemischen in Steam-Crackern, katalytischen Crackern, Isomerisierungs- oder Dehydrierunganlagen gewonnen werden. Sie können als solche mit einer größeren Breite an C-Atomzahlen erfindungsgemäß eingesetzt werden oder als engere Schnitte, die im wesentlichen beispielsweise $C_5$-Kohlenwasserstoffe oder $C_6$-Kohlenwasserstoffe mit nur geringen Anteilen der anschließenden Homologen enthalten. Typische Zusammensetzungen für $C_5$-Schnitte aus Steam-Crackern oder katalytischen Crackern sind etwa wie folgt:

| Stoff, Gew.-% (ca.) | Steamcracker | | | Cat. Cracker | | |
|---|---|---|---|---|---|---|
| C₄/Leichte | 1 | - | 5 | 1 | - | 5 |
| n-/i-Pentan | 20 | - | 30 | 30 | - | 35 |
| n-Pentene | 15 | - | 20 | 25 | - | 30 |
| 3-Methyl-buten-1 | 0,5 | - | 3 | 0,5 | - | 2 |
| 2-Methyl-buten-1 | 5 | - | 10 | 7 | - | 11 |
| 2-methyl-buten-2 | 8 | - | 17 | 15 | - | 20 |
| Cyclopentan | 4 | - | 6 | 1 | - | 3 |
| Cyclopenten | 20 | - | 27 | 2 | - | 5 |
| Diolefine/Acetylene | 0,5 | - | 5 | 0,5 | - | 5 |

Als Alkanole für die Herstellung der erfindungsgemäßen Kraftstoff-Komponenten seien beispielsweise primäre oder sekundäre, bevorzugt primäre Alkanole mit 1-8 C-Atomen, bevorzugt 1-4, besonders bevorzugt 1-2 C-Atomen genannt. Ganz besonders bevorzugt ist der Einsatz von Methanol. Die Alkanole werden in einer Menge von 0,7-4 Mol Alkanol/Mol Gesamtmenge an tert.-Olefinen im rohen Kohlenwasserstoffgemisch eingesetzt; bevorzugt ist ein Verhältnis von 0,8-2,5 : 1, besonders bevorzugt ein Verhältnis von 1-2 : 1.

Wasserstoff wird in einer Menge eingesetzt, die mindestens äquimolar ist zur Gesamtmenge der Gum-Bildner, beispielsweise in einer Menge von 1-2 Mol/Mol. Wasserstoff kann in reiner oder technischer Form eingesetzt werden. Wirtschaftlich vorteilhaft kann ein in petrochemischen Anlagen anfallender, mit CH₄ und/oder N₂ vergesellschafteter Wasserstoff eingesetzt werden. Die H₂-Menge in solchen reinen oder technischen Wasserstoffen beträgt 70 bis 100 % H₂, in H₂-haltigen Restgasen von petrochemischen Anlagen vielfach etwa 80-90 % H₂.

Das Herstellungsverfahren der erfindungsgemäßen Kraftstoff-Komponenten wird in Gegenwart eines makroporösen oder gelförmigen sauren Kationenaustauschers in der H⁺-Form mit einem Vernetzungsgrad von 2-65 %, bevorzugt 8-25 % und einer spezifischen Oberfläche von 5-750 m²/g, bevorzugt 50-250 m²/g trockenen Austauscherharzes durchgeführt, der mit einem oder mehreren der unten näher bezeichneten Metalle in elementarer Form belegt ist. Makroporöse oder gelförmige saure Kationenaustauscher sind dem Fachmann bekannt und können beispielsweise durch Copolymerisation von Vinylmonomeren und Divinylvernetzern, gegebenenfalls in Gegenwart von Lösungsmitteln oder durch Kondensation von Phenol und Formaldehyd hergestellt.

Vinylmonomere sind beispielsweise Styrol oder Acrylsäureester; Divinylvernetzer ist beispielsweise Divinylbenzol. Saure Gruppen solcher Kationenaustauscher. sind beispielsweise Carboxylgruppen, Phosphonsäuregruppen oder Sulfonsäuregruppen. Bevorzugt eingesetzt werden stark saure, Sulfonsäuregruppen enthaltende Styrol-Divinylbenzol-Polymerisate, die unter verschiedenen Bezeichnungen handelsüblich sind.

Der mittlere Porenradius der Kationenaustauscher kann beispielsweise in Grenzen von 5-120 nm (50-1200 Å), vorzugsweise 7-50 nm (70-500vÅ), variieren. Solche Kationenaustauscher können beispielsweise als Perlpolymerisate Korngrößen von 0,1-2 mm, als Pulverharz Korngrößen von 10-100 µm aufweisen.

Die Kationenaustauscher werden in der H⁺-Form eingesetzt. nachdem sie mit 0,001-10 g, bevorzugt 0,2 bis 3 g, bezogen auf 1l trockenen Kationenaustauscher, eines oder mehrerer Metalle der VI. und/oder VII. und/oder VIII. Nebengruppen des Periodensystems der Elemente (Mendelejev) in elementarer Form belegt worden sind.

Als solche Metalle seien beispielsweise genannt: Chrom, Molybdän, Wolfram, Mangan, Rhenium, Eisen, Cobalt, Nickel, Ruthenium, Rhodium, Palladium, Osmium, Iridium und Platin. Bevorzugt seien genannt: Palladium, Platin, Rhenium, Molybdän oder Nickel, ganz besonders bevorzugt sind Palladium, Platin und Nickel, In weiterhin bevorzugter Weise wird jeweils nur eines der genannten Metalle eingesetzt.

Die Belegung der Kationenaustauscher mit einem oder mehreren der genannten Metalle kann beispielsweise so erfolgen, daß man ein nicht-komplexes, kationisches Salz dieser Metall mit dem Kationenaustauscher in der H⁺-Form in an sich bekannter Weise zusammenbringt.

Gegenbenenfalls wird die hierbei freiwerdende Säure durch geeignete alkalisch reagierende Verbindungen, beispielsweise Alkalihydroxid-Lösungen neutralisiert. Die Menge des aufzubringenden Metallsalzes wird berechnet oder durch einfache Vorversuche bestimmt, so daß die gewünschte Menge an Metall in elementarer Form auf den Kationenaustauscher aufgebracht wird.

Der metalldotierte Kationenaustauscher wird neutral gewaschen, getrocknet, beispielsweise bei 80-100°C im Vakuum, und anschließend zur Überführung der aufgebrachten Metalle in den elementaren Zustand mit Wasserstoff behandelt, beispielsweise bei 2-50 bar, vorzugsweise 20-30 bar und einer Temperatur von 50-140°C, vorzugsweise 80-120°C. Grundsätzlich können auch andere Reduktionsmittel, wie Hydrazin oder Formaldehyd benutzt werden.

Der metalldotierte Kationenaustauscher wird einer Belastung von a = 0,1-100, bevorzugt 0,3-20, besonders bevorzugt 0,5-5 unterworfen. Das Zeichen "a" bedeutet hierbei kg rohes, tert.-Olefine enthaltendes Kohlenwasserstoffgemisch pro kg Kationenaustauscher pro Stunde.

Die Herstellung der erfindungsgemäßen Kraftstoff-Komponenten erfolgt bei einer Temperatur von 30-140°C, bevorzugt 35-110°C, besonders bevorzugt 40-90°C. Hierbei wird ein Druck eingestellt, bei dem das Reaktionsgemisch mit Ausnahme des nicht gelösten $H_2$ mindestens teilweise flüssig ist. Der Zusammenhang zwischen der gewählten Arbeitstemperatur und einem solchen einzustellenden Druck ist dem Fachmann geläufig.

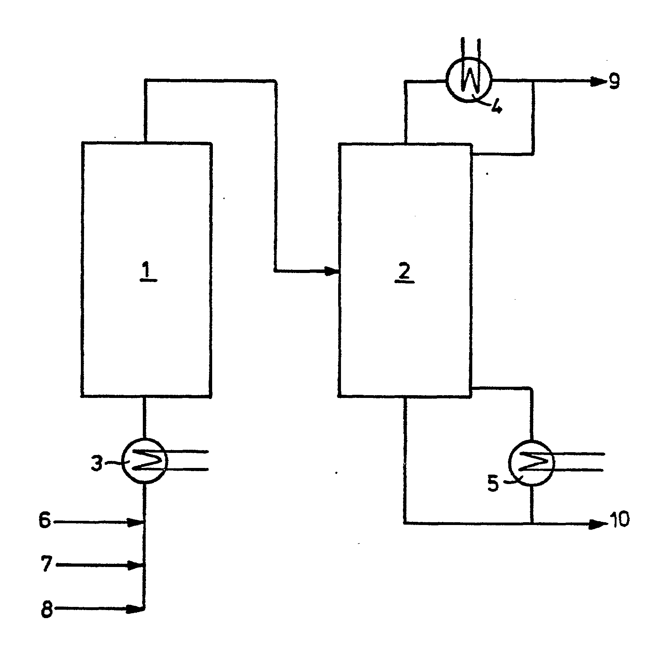

Zur Herstellung der erfindungsgemäßen Kraftstoff-Komponenten kann beispielsweise wie folgt verfahren werden, wobei auf die beigefügte Zeichnung Bezug genommen wird:

Das rohe Kohenwasserstoffgemisch (6), der (die) Alkanol(e) (7) und $H_2$ (8) als Einsatzstoffströme werden nach Durchlaufen des Vorwärmers (3) dem Reaktor (1) mit dem metallbeladenen Kationenaustauscher zugeführt und dort umgesetzt. Nach Verlassen von (1) gelangt das Umsetzungsgemisch in die Stabilisierungskolonne (2), in der das Restgas, beispielsweise überschüssiger Wasserstoff und gegebenenfalls vorhandenes $CH_4$ oder $N_2$ über Kopf als Strom (9) abgezogen wird. Im Kühler (4) kondensierbare Stoffe werden nach (2) zurückgeleitet. (2) wird durch einen Umlauferhitzer (5) beheizt. Ein Teil des umlaufenden Sumpfproduktes wird als Gum-freie, Alkyl-tert.-alkyl-ether enthaltende Kraftstoff-Komponente (10) abgezogen.

Die erfindungsgemäßen Kraftstoff-Komponenten sind durch einen Gehalt an Octanzahl-erhöhenden Alkyl-tert.-alkyl-ethern ausgezeichnet.

Beispielsweise enthält eine Kraftstoff-Komponente aus einem $C_5$Schnitt eines Steam-Crackers oder katalytischen Crackers, wenn die Umsetzung mit Methanol vorgenommen wurde, tert.-Amyl-methyl-ether (TAME). Der Umfang der Octanzahl-Erhöhung ist selbstverständlich in einer dem Fachmann geläufigen Weise von der Menge der veretherten tert.-Olefine abhängig. Ein weiterer Vorteil der erfindungsgemäßen Kraftstoff-Komponenten ist die wünschenswerte Verringerung der Sensitivity, d.h. die Verringerung des Abstandes zwischen der Motor-Octanzahl (MOZ) und der Research-Octanzahl (ROZ) und die Verbesserung ihrer Farbzahl. So wird bei einem Gehalt an veretherbaren Olefinen im Ausgangskohlenwasserstoffgemisch von beispielsweise 10-30 Gew.-% ein Gehalt an Alkyl-tert.-alkyl-ether in den Kraftstoff-Komponenten von etwa 12-42 Gew.-% erreicht.

Die Sensitivity sinkt von etwa 18-20 auf etwa 13-15, und dei Farbzahl nach APHA ist stets unter 8, vielfach unter 5 und kann Werte unter 4 erreichen.

Insbesondere enthalten die erfindungsgemäßen Kraftstoff-Komponenten nur wenig Gum-Bildner, so daß die sichere Einhaltung und Unterschreitung der in DIN 51607 und DIN 51600 bzw. in der ASTM-Norm für Automotive Gasoline festgelegten Höchstmenge für Gum (als Abdampfrückstand) von 5 mg/100 ml Kraftstoff gewährleistet ist.

Durch den Zusatz von Wasserstoff zur Herstellung der Kraftstoff-Komponenten und durch die damit verhinderte Gum-Bildung wird weiterhin die Standzeit des Kationenaustauschers wesentlich verlängert. Diese Standzeit beträgt beispielsweise ohne Zusatz von $H_2$ etwa 8 Monate, mit Zusatz von $H_2$ mindestens 2 Jahre.

Selbstverständlich können die erfindungsgemäßen Gumfreien, Alkyl-tert.-alkyl-ether enthaltenden Kraftstoff-Komponenten, falls dies gewünscht wird, in einer dem Fachmann bekannten Weise destillativ aufgetrennt werden, beispielsweise um die hierbei erhältlichen Teile der Kraftstoff-Komponeten separat und gezielt anderen Kraftstoffen oder anderen Verwendungen zuzuführen. Beispielsweise könnte man einer solchen destillativen Auftrennung folgende Anteile der erfindungsgemäßen Kraftstoff-Komponenten entnehmen: Das Restgas und nicht umgesetzte Kohlenwasserstoffe als Kopfstrom: den hauptsächlich erhaltenen Alkyl-tert.-alkyl-ether (beispielsweise TAME aus einem $C_5$-Schnitt); einen Gum-freien Sumpfstrom, der höhere Ether und andere Höhersieder enthält.

Die folgenden Beispiele illustrieren die Erfindung, ohne sie einzuschränken.

Beispiel 1

(Herstellung eines erfindungsgemäß einzusetzenden Kationenaustauschers)

Dem handelsüblichen Kationenaustauscher LEWATIT SPC 118 (Styrol-Divinyl-Copolymer mit Sulfonsäuregruppen der Bayer AG) wurde in der wasserfeuchten $H^+$-Form soviel Palladiumacetat angeboten, daß ein g Pd pro Liter trockenes Harz nach Reduktion mit $H_2$ auf dem Kationenaustauscher vorhanden waren. Die bei der Behandlung mit Palladiumacetat freigesetzte Säure wurde mit 1 gew.-%iger NaOH neutralisiert. Der neutralgewaschene Kationenaustauscher wurde 24 Stunden bei 100°C im Vakuum einer Wasserstrahlpumpe getrocknet. Das auf dem Kationenaustauscher befindliche Palladium wurde bei 90-100°C und 20-25 bar H₂-Druck innerhalb von 48 Stunden zum Metall reduziert.

Beispiel 2

Der nach Beispiel 1 erhaltene Kationenaustauscher wurde in einen temperierbaren Durchlaufreaktor mit 20 mm lichter Weite und Temperaturmeß-Stellen im Abstand von jeweils 100 mm eingefüllt. Als rohes Kohlenwasserstoffgemisch wurde der C₅-Strom eines Steam-Crackers (sogenannter Aromaten-Vorlauf) mit einem Gehalt von 14,5 Gew.-% 2-Methylbuten (2), 3,8 Gew.-% 2-Methylbuten-(1) und 0,5 Gew.-% 3-Methylbuten-(1) eingesetzt.

Dieser Stoffstrom hatte eine MOZ = 76 und eine ROZ = 91 und einen Siedebereich von 35-65°C. Die Belastung des Pd-beladenen Kationenaustauschers wurde zu a = 1 und der Druck auf 10 bar eingestellt ; die Temperatur wurde zwischen 70 und 75°C gehalten. Das Kohlenwasserstoffgemisch wurde mit der stöchiometrischen Menge an Methanol (bezogen auf die tert.-Amylene) in einer Mischkammer vor dem Reaktor gemischt und vorgewärmt. Die zugegebene Wasserstoffmenge wurde so eingestellt, daß sie eine Gasbelastung des Kationenaustauschers von 80 l H₂ pro Liter Kationenaustauschers pro Stunde ergab.

Als Reaktionsprodukt wurde eine farblose Kraftstoff-Komponente mit 17,6 Gew.-% TAME erhalten. Diese Kraftstoff-Komponente hat einen Abdampfrückstand von 1 mg/100 ml, eine Farbzahl nach APHA von ( 5, eine MOZ (Clear) = 80 und ein ROZ (clear) = 93.

Beispiel 3

Der Versuch wurde wie in Beispiel 2 durchgeführt, jedoch ohne Zugabe von H₂. Das ebenfalls 17,6 Gew.-% TAME enthaltende Reaktionsprodukt ist jedoch gelb gefärbt, besitzt eine Farbzahl nach APHA von ca. 3000 und einen Abdampfrückstand von 21 mg/100 ml. Die Octanzahlwerte gleichen denen aus Versuch 2.

**Patentansprüche**

1. Alkyl-tert.-alkyl-ether enthaltende Gum-freie Kraftstoff-Komponenten, dadurch herstellbar, daß man tert.-Olefine und Gum-Bildner enthaltende rohe Kohlenwasserstoffgemische gleichzeitig mit Alkanolen und Wasserstoff, der in einer Menge eingestzt wird, die zur Gesamtmenge der Gum-Bildner mindestens äquimolar ist, an einem makroporösen oder gelförmigen Kationenaustauscher in der H⁺-Form, der 0,001 – 10 g eines oder mehrerer Metalle der VI. und/oder VII. und/oder VIII. Nebengruppe des Periodensystems der Elemente in elementarer Form pro Liter trockenen Kationenaustauschers enthält und einen Vernetzungsgrad von 2–65% sowie eine spezifische Oberfläche von 5–750 m²/g trockenes Austauscherharz aufweist, in flüssiger Phase bei 30 bis 140°C umsetzt.

2. Kraftstoff-Komponenten nach Anspruch 1, dadurch gekennzeichnet, daß der Kationenaustauscher als Metall Palladium, Platin, Rhenium, Molybdän oder Nickel enthält.

3. Kraftstoff-Komponenten nach Ansprüchen 1 bis 2, dadurch gekennzeichnet, daß der Kationenaustauscher als Metall Palladium, Platin oder Nickel, jeweils für sich allein, enthält.

4. Kraftstoff-Komponenten nach Ansprüchen 1-3, dadurch gekennzeichnet, daß man die Umsetzung bei einer Belastung des Kationenaustauschers von 0,1-100 kg, bevorzugt 0,3-20 kg, besonders bevorzugt 0,5-5 kg des rohen Kohlenwasserstoffstromes pro kg Kationenaustauscher pro Stunde durchführt.

5. Kraftstoff-Komponenten nach Ansprüchen 1-4, dadurch gekennzeichnet, daß Wasserstoff in einer Menge von 1-2 Mol, bevorzugt 1-1,2 Mol pro Mol der Gesamtmenge an Gum-Bildner eingesetzt wird.

6. Kraftstoff-Komponenten nach Ansprüchen 1-5, dadurch gekennzeichnet, daß technischer Wasserstoff eingesetzt wird.

7. Kraftstoff-Komponenten nach Ansprüchen 1-6, dadurch gekennzeichnet, daß das Reaktionsgemisch mit Ausnahme des nicht gelösten Wasserstoffs mindestens teilweise in flüssiger Phase vorliegt.

8. Kraftstoff-Komponenten nach Ansprüchen 1-7, dadurch gekennzeichnet, daß die Alkanolmenge 0,7-4 Mol bevorzugt 0,8-2,5 Mol, besonders bevorzugt 1-2 Mol pro Mol der Gesamtmenge an tert.-Olefinen beträgt.

9. Kraftstoff-Komponenten nach Ansprüchen 1-8, dadurch gekennzeichnet, daß als Alkanol ein primärer oder sekundärer mit 1-8, bevorzugt 1-4 C-Atomen eingesetzt wird.

10. Kraftstoff-Komponenten nach Ansprüchen 1-9, dadurch gekennzeichnet, daß als Alkanol Methanol oder Ethanol eingesetzt werden.

**Claims**

1. Alkyl tert-alkyl ether-containing gum-free motor fuel components which can be prepared by reacting tert-olefin- and gum former-containing crude hydrocarbon mixtures in the liquid phase at 30 to 140°C with, at the same time, alkanols and hydrogen, which is used in an amount which is at least equimolar with the total amount of gum formers, on a macroporous or gel-like cation exchanger in the H⁺-form which contains 0.001–10 g of one or more metals of subgroup VI and/or VII and/or VIII of the periodic table of the elements in elemental form per litre of dry cation exchanger and has a degree of crosslinking of 2–65% and a specific surface of 5–750 m²/g of dry exchange resin.

2. Motor fuel components according to claim 1, characterized in that the cation exchanger contains as metal palladium, platinum, rhenium, molybdenum or nickel.

3. Motor fuel components according to claims 1 to 2, characterized in that the metal contained by the cation exchanger is palladium, platinum or nickel, in each case, alone.

4. Motor fuel components according to claims 1 – 3, characterized in that the reaction is carried out under a cation exchanger loading of 0.1–100 kg, preferably 0.3–20 kg, particularly preferably 0.5–5 kg of the crude hydrocarbon stream per kg of cation exchanger per hour.

5. Motor fuel components according to claims 1 – 4, characterized in that hydrogen is used in an amount of 1–2 mol, preferably 1–1.2 mol per mol of the total amount of gum former.

6. Motor fuel components according to claims 1 – 5, characterized in that industrial hydrogen is used.

7. Motor fuel components according to claims 1 – 6, characterized in that the reaction mixture, with the exception of the undissolved hydrogen, is present at least partly in the liquid phase.

8. Motor fuel components according to claims 1 – 7, characterized in that the amount of alkanol is 0.7–4 mol, preferably 0.8–2.5 mol, particularly preferably 1–2 mol per mol of the total amount of tert-olefins.

9. Motor fuel components according to claims 1 – 8, characterized in that the alkanol used is a primary or secondary alkanol having 1–8, preferably 1–4 carbon atoms.

10. Motor fuel components according to claims 1 – 9, characterized in that the alkanol used is methanol or ethanol.

**Revendications**

1. Composants de carburant sans gomme contenant des éthers d'alkyle-alkyle tertiaire, pouvant être fabriqués en faisant réagir en phase liquide à une température de 30 à 140°C des mélanges bruts d'hydrocarbures contenant des oléfines tertiaires et des formateurs de gomme avec simultanément des alcanols et de l'hydrogène introduit en quantité au moins équimolaire par rapport à la quantité totale des formateurs de gomme, sur un échangeur de cations sous forme H+, à grands pores ou sous forme de gel, qui contient sous forme élémentaire 0,001 à 10 g d'un ou plusieurs métaux des sous-groupes VI et/ou VII et/ou VIII du système périodique des éléments par litre d'échangeur cationique sec et présente un degré de réticulation de 2 à 65% et une surface spécifique de 5 à 750 m²/g de résine échangeuse sèche.

2. Composants de carburant selon la revendication 1, caractérisés en ce que l'échangeur de cations contient comme métal du palladium, du platine, du rhénium, du molybdène ou du nickel.

3. Composants de carburant selon les revendications 1 à 2, caractérisés en ce que l'échangeur de cations contient comme métal du palladium, du platine ou du nickel, chaque fois seul.

4. Composants de carburant selon les revendications 1 à 3, caractérisés en ce que l'on effectue la réaction pour une charge de l'échangeur de cations de 0,1 à 100 kg, de préférence de 0,3 à 20 kg, de préférence particulière de 0,5 à 5 kg de courant brut d'hydrocarbures par kg d'échangeur cationique par heure.

5. Composants de carburants selon les revendications 1 à 4, caractérisés en ce que l'hydrogène est introduit en quantité de 1 à 2 moles, de préférence de 1 à 1,2 mole par mole de quantité totale de formateurs de gomme.

6. Composants de carburant selon les revendications 1 à 5, caractérisés en ce qu'on utilise de l'hydrogène technique.

7. Composants de carburant selon les revendications 1 à 6, caractérisés en ce que le mélange réactionnel se trouve au moins partiellement en phase liquide, à l'exception de l'hydrogène non dissous.

8. Composants de carburant selon les revendications 1 à 7, caractérisés en ce que la quantité d'alcanol est de 0,7 à 4 moles, de préférence de 0,8 à 2,5 moles et de préférence particulière de 1 à 2 moles par mole de quantité totale d'oléfines tertiaires.

9. Composants de carburant selon les revendications 1 à 8, caractérisés en ce que l'on utilise comme alcanol un alcanol primaire ou secondaire avec 1 à 8, de préférence 1 à 4 atomes de carbone.

10. Composants de carburant selon les revendications 1 à 9, caractérisés en ce qu'on emploie comme alcanol du méthanol ou de l'éthanol.